# EUROPÄISCHE PATENTANMELDUNG

(11) **EP 3 020 453 A1**
(43) Veröffentlichungstag der Anmeldung: **18.05.2016**
(21) Anmeldenummer: 14193398.6
(22) Anmeldetag: 17.11.2014
(51) Int. Cl.: A61Q 3/02, A61K 8/87

(54) **Polyurethanharnstoffe für wässrige Nagellackzusammensetzungen**

(71) Anmelder: Covestro Deutschland AG, 51373 Leverkusen (DE)
(72) Erfinder: VIALA, Sophie, 50935 Köln (DE); DÖRR, Sebastian, 40593 Düsseldorf (DE); NAZARAN, Panthea, 51061 Köln (DE)
(74) Vertreter: Levpat

(57) **Zusammenfassung**

Die Erfindung betriff eine wässrige Nagellackzusammensetzung, dadurch gekennzeichnet, dass diese eine wässrige Dispersion eines kationisch hydrophilierten Polyurethanharnstoffs und ≤ 10 Gew.-% an organischen Lösemitteln, bezogen auf die gesamte Nagellackzusammensetzung, enthält. Ebenfalls betrifft die Erfindung ein Verfahren zur Herstellung der wässrigen Nagellackzusammensetzung. Weiterhin betrifft die Erfindung die Verwendung von wässrige Dispersion eines kationisch hydrophilierten Polyurethanharnstoffs in wässrigen Nagellackzusammensetzungen, sowie ein Verfahren zur Beschichtung von Nägeln unter Verwendung der wässrigen Nagellackzusammensetzungen.

## Beschreibung

Die vorliegende Erfindung betrifft eine wässrige Nagellackzusammensetzung, enthaltend eine wässrige Polyurethanharnstoffdispersion, sowie ein Verfahren zur Herstellung der wässrigen Nagellackzusammensetzung. Weiterhin betrifft die Erfindung die Verwendung von wässrigen Po-lyurethanharnstoffdispersionen in wässrigen Nagellackzusammensetzungen, sowie ein Verfahren zur Beschichtung von Nägeln unter Verwendung der wässrigen Nagellackzusammensetzungen.

Nagellacke, wie sie heutzutage zur Anwendung kommen, werden nahezu ausschließlich auf Basis lösemittelhaltiger physikalisch trocknender Bindemittel hergestellt. Insbesondere wird überwiegend Nitrocellulose als Hauptbestandteil im lösemittelhaltigen Bindemittel verwendet.

Aufgrund der aufkommenden Diskussion zur Reduzierung von flüchtigen organischen Lösemitteln im Kosmetikbereich, gibt es ein großes Interesse an der Verringerung oder gar an der gänzlichen Vermeidung von Lösemittelanteilen in konventionellen Nagellacken.

Nitrocellulose selbst ist in Wasser praktisch nicht löslich. Erst durch Modifikation des Polymergerüstes wie z.B. der Einbringung von hydrophilen Seitengruppen kann eine Wasserlöslichkeit hergestellt werden. Durch Veränderung des Polymergerüstes werden jedoch auch die für die Anwendung im Nagellackbereich zuvor positiven Eigenschaften der Nitrocellulose wie z.B. der hohe Glanz negativ beeinflusst.

Aus diesem Grunde wird versucht, auf andere Polymersysteme auszuweichen, die eine Wasserlöslichkeit neben den anderen geforderten Eigenschaften wie Filmbildung, mechanische Eigenschaf-ten, etc. aufweisen.

So werden z.B. in US 5955063 wässrige Acrylat-Bindemittel für die Herstellung von wasserbasierten Nagellacken beschrieben.

Von großem Nachteil ist bei diesen wässrigen Bindemitteln jedoch, dass wesentliche Eigenschaften wie Wasserfestigkeit, Härte, Glanz und Trocknungszeit nicht den praktischen Anforderungen genügen.

Darüber hinaus beschreibt US 5637292 die Verwendung von wässrigen Acrylatpolymeren mit einem Anteil an Acrylatmonomeren, welche mittels UV-Licht nach Formulierung des Lagelackes zur Reaktion gebracht werden und somit eine sehr schnelle Trocknung/Härtung aufweisen. Nachteilig bei diesen Systemen ist jedoch die Anwesenheit von Acrylat-monomeren, welche aus Sicht der Anwendungshygiene als bedenklich eingestuft werden müssen. Darüber hinaus kann die Einwirkung von UV-Licht gewebeschädigend sein und sollte daher vermieden werden.

Im Stand der Technik, beispielsweise in den Patentanmeldungen EP 0391322 A1, EP 0418469 A1 und EP 0423471 A2 wird die Verwendung von wässrigen Polyurethandispersionen in wasserbasierten Nagellacken beschrieben.

In der WO2007/115675 A1 werden zudem wässrige Nagellacke mit Nitrocellulose-haltigen, wässrigen Polyurethandispersionen offenbart.

Auch die WO 2012/061265 A1 befasst sich mit Nitrocellulose freien Nagellacken, zu deren Herstellung unter anderem wässrige Polyurethandispersionen eingesetzt werden.

Die bisher eingesetzten wässrigen Polyurethandispersionen basieren jedoch auf Polyurethanen, die um ihre Dispergierbarkeit in Wasser sicherzustellen mit anionischen Gruppen modifiziert sind. Die damit erhaltenen Nagellacke weisen jedoch eine schlechte Beständigkeit in Wasser, sowie eine zu niedrige Härte auf.

Aufgabe der vorliegenden Erfindung war es daher, neue wässrige Bindemittel für die Herstellung von wasserbasierten Nagellacken zur Verfügung zu stellen, die eine verbesserte Härte und Wasserbeständigkeit aufweisen.

Diese Aufgabe wurde erfindungsgemäß gelöst durch eine wässrige Nagellackzusammensetzung, die eine wässrige Dispersion eines kationisch hydrophilierten Polyurethanharnstoffs und ≤ 10 Gew.-% an organischen Lösemitteln, bezogen auf die gesamte Nagellackzusammensetzung, enthält.

Die erfindungsgemäßen wässrigen Nagellackzusammensetzungen weisen überraschend nach Trocknung eine besonders gute Härte und Wasserbeständigkeit im Vergleich zu den wässrigen Nagellacken nach dem Stand der Technik auf.

Ein weiterer Gegenstand der Erfindung ist ein Verfahren zur Herstellung einer wässrigen Nagellackzusammensetzung, dadurch gekennzeichnet, dass eine wässrige Dispersion eines kationisch hydrophilierten Polyurethanharnstoffs und ≤ 10 Gew.-% an organischen Lösemitteln, bezogen auf die gesamte Nagellackzusammensetzung, eingesetzt werden.

Ebenfalls Gegenstand der Erfindung ist die Verwendung von wässrigen Dispersionen von kationisch hydrophilierten Polyurethanharnstoffen in wässrigen Nagellackzusammensetzungen, die ≤ 10 Gew.-% an organischen Lösemitteln, bezogen auf die gesamte Nagellackzusammensetzung, enthalten.

Gegenstand der Erfindung ist auch ein Verfahren zur kosmetischen Beschichtung von Nägeln, dadurch gekennzeichnet, dass eine erfindungsgemäße wässrige Nagellackzusammensetzung auf Nägel aufgetragen wird.

Erfindungsgemäß wird unter einem kationisch hydrophilierten Polyurethanharnstoff ein Po-lyurethanharnstoff verstanden, der an das Polymergerüst gebundene, kationische oder potentiell kationische Gruppen aufweist. Unter potentiell kationischen gruppen sind solche Gruppen zu verstehen, die durch chemische Reaktion, insbesondere durch Neutralisation in eine kationische Gruppe überführt werden können.

Bevorzugt weisen die in der erfindungsgemäßen Nagellackzusammensetzung enthaltenen Po-lyurethanharnstoffe einen Gehalt an kationischen und/oder potentiell kationischen Gruppen von ≥ 0,2 und ≤ 5 Milliequivalenten pro g Polymer, besonders bevorzugt von ≥ 0,5 und ≤ 2 Milliequivalenten pro g Polymer und ganz besonders bevorzugt von ≥ 0,6 und ≤ 1 Milliequivalenten pro g Polymer auf.

Polyurethanharnstoffe im Sinne der Erfindung sind polymere Verbindungen, die mindestens zwei bevorzugt mindestens drei Urethan-Gruppen-haltige Wiederholungseinheiten 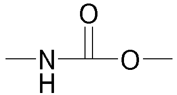 und zudem auch Harnstoff-Gruppen-haltige Wiederholungseinheiten aufweisen: 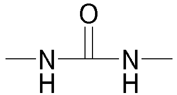 Die Harnstoffgruppen werden dabei bevorzugt durch die Reaktion von ioscyanatfunktionellen Polyurethanpräpolymeren mit Aminogruppen aufweisenden Verbindungen gebildet

In einer bevorzugten Ausführungsform der Erfindung ist der Polyurethanharnstoff aufgebaut aus
a) wenigstens einem aliphatischen, araliphatischen und/oder cycloaliphatischen Polyisocyanat,
b) wenigstens einem Polyol mit einem zahlenmittleren Molekulargewicht Mn ≥ 400 und ≤ 6000 g/mol und einer Hydroxylfunktionalität von ≥ 1,5 und ≤ 4,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist.

Der Polyurethanharnstoff kann auch weitere Aufbaukomponenten enthalten, bevorzugt ist er jedoch ausschließlich aus den Komponenten a) bis f) und besonders bevorzugt ausschließlich aus den Komponenten a) bis e) aufgebaut.

Gegenüber Isocyanatgruppen reaktive Gruppen sind im Sinne dieser Erfindung insbesondere primäre und sekundäre Aminogruppen, Hydroxylgruppen und/oder Thiolgruppen.

Das zahlenmittlere Molekulargewicht wird im Rahmen dieser Anmeldung stets bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Die Komponente a) umfasst wenigstens ein aliphatisches, araliphatisches und/oder cycloaliphatisches Polyisocyanat.

Als Komponente a) geeignete Verbindungen sind beispielsweise 1,4-Butylendiisocyanat, 1,5-Pentamethy¬lendiisocyanat (PDI), 1,6-Hexamethy-lendiisocyanat (HDI), Isophorondiisocyanat (IPDI), 2,2,4- und/oder 2,4,4-Trimethyl-hexa¬methy¬len¬diiso¬cyanat, die isomeren Bis-(4,4'-isocyanatocyclo¬hexyl)¬methane oder deren Mischungen beliebigen Isomeren¬gehalts (H12-MDI), 1,4-Cyclohexylendi-isocyanat, 4-Isocyanatomethyl-1,8-octandiisocyanat (Nonantriisocyanat), 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin¬diisocyanate) mit C1-C8-Alkylgruppen, sowie deren Gemische.

Neben den vorstehend genannten Polyisocyanaten können anteilig auch modifizierte Diisocyanate oder Triisocyanate mit Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit einer mittleren NCO-Funktionalität von ≥ 2 und ≤ 4, bevorzugt > 2 bis 2,6 und besonders bevorzugt ≥ 2 und ≤ 2,4.

Bevorzugt werden als Komponente a) HDI, H12-MDI und/oder IPDI eingesetzt.

Besonders bevorzugt umfasst die Komponente a) ≥ 90 Gew.-%, weiterhin bevorzugt ≥ 95 Gew.-% und insbesondere bevorzugt 100 Gew.-% IPDI, bezogen auf die Gesamtmasse der Komponente a). Der Anteil der Komponente a) am Polyurethanharnstoff ist bevorzugt ≥ 5 und ≤ 75 Gew.-%, besonders bevorzugt ≥ 10 und ≤ 65 Gew.-% und ganz besonders bevorzugt > 20 und ≤ 55 Gew.-%, bezogen auf das Gesamtgewicht des Polyurethanharnstoffs. Die Komponenten a) bis f) ergänzen sich dabei zu 100 Gew.-%.

Die Komponente b) umfasst wenigstens ein Polyol mit einem zahlenmittleren Molekulargewicht Mn ≥ 400 und ≤ 6000 g/mol und einer Hydroxylfunktionalität von ≥ 1,5 und ≤ 4.

Bevorzugt weist die Komponente b) ein zahlenmittleres Molekulargewichte von ≥ 600 und ≤ 4000 g/mol, besonders bevorzugt von ≥ 800 und ≤ 3000 g/mol und / oder eine mittlere OH-Funktionalitäten von ≥ 1,8 und ≤ 3 und besonders bevorzugt von ≥ 1,9 und ≤ 2,lauf.

Als Komponente b) geeignete Verbindungen sind insbesondere Polyesterpolyole, Polyetherpolyole, Polycarbonatpolyole, Polyetherpolycarbonatpolyole und/oder Polyesterpolycarbonatpolyole.

Bevorzugt umfasst die Komponente b) wenigstens ein Polyesterpolyol, besonders bevorzugt ist die Komponente b) ausgewählt aus Polyesterpolyolen.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester.. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Der Anteil der Komponente b) am Polyurethanharnstoff ist bevorzugt > 5 und ≤ 70 Gew.-%, besonders bevorzugt ≥ 10 und ≤ 60 Gew.-% und ganz besonders bevorzugt ≥ 20 und ≤ 50 Gew.-%, bezogen auf das Gesamtgewicht des Polyurethanharnstoffs. Die Komponenten a) bis f) ergänzen sich dabei zu 100 Gew.-%.

In einer bevorzugten Ausführungsform der Erfindung umfasst die Komponente b) wenigstens ein Polyesterpolyol b1), das als Aufbaukomponente wenigstens eine aromatische Dicarbonsäure und/oder das entsprechende Carbonsäureanhydrid enthält. Geeignete aromatische Dicarbonsäuren oder Carbonsäureanhydride sind dabei insbesondere Phthalsäure, Isophthalsäure und/oder Terephthalsäure, die korrespondierenden Anhydride, sowie Gemische der genannten Verbindungen.

Bevorzugt beträgt der Anteil an aromatischen Dicarbonsäuren und/oder deren Anhydriden im Polyesterpolyol ≥ 35 Gew.-%, besonders bevorzugt ≥ 45 Gew.-% und ganz besonders bevorzugt ≥ 70 Gew.-%, bezogen auf die Gesamtmasse des Polyesterpolyols.

Es können dabei Mischungen aus aliphatischen und aromatischen Dicarbonsäuren und/oder deren Anhydriden als Säurekomponente bei der Herstellung eingesetzt werden, bevorzugt werden jedoch ausschließlich aromatische Dicarbonsäuren und/oder Anhydride und keine aliphatischen Carbonsäuren und/oder Anhydride als Säurekomponente eingesetzt.

Bevorzugt enthält das Polyesterpolyol b1) als Diolkomponente Ethylenglykol, 1,4-Butandiol und/oder 1,6-Hexandiol, besonders bevorzugt Ethylenglykol.

Bevorzugt ist das Polyesterpolyol b1) aufgebaut ausschließlich aus Ethylenglykol, 1,4-Butandiol und/oder 1,6-Hexandiol und aromatischen Dicarbonsäure und/oder deren Anhydriden, besonders bevorzugt aus Ethylenglykol und Phthalsäure und/oder Phthalsäureanhydrid.

Bevorzugt weisen die eingesetzten Polyesterpolyole b1) eine amorphe Struktur auf.

Bevorzugt weisen die Polyesterpolyole b1) dabei einen Glasübergangspunkt Tg, bestimmt mittels DSC, von ≥ -25°C und ≤ 80°C, besonders bevorzugt ≥ 0°C und ≤ 60°C und ganz besonders bevorzugt > 10°C und ≤ 55°C auf.

Im Rahmen der Erfindung werden die Glasübergangstemperatur Tg, mittels dynamischer Differenzkalorimetrie (DSC) in Anlehnung an die DIN EN 61006, Verfahren A, bestimmt, wobei ein DSC Gerät (Kalorimeter Pyris Diamond DSC von Perkin-Elmer) verwendet wird, das zur Bestimmung von T_{g} mit Indium und Blei kalibriert ist. Es werden 10 mg der zu untersuchenden Substanz in einen verschließbaren Aluminium-Tiegel eingewogen und dieser verschlossen. Es werden drei unmittelbar aufeinander folgende Durchläufe einer Aufheizungen von -100°C bis +150°C, Heizrate 20 K/min, mit anschließender Abkühlung Kühlrate 320 K/min vorgenommen und die dritte Aufheizkurve zur Bestimmung der Werte verwendet. Als T_{g} wird die Temperatur bei der halben Höhe einer Glasübergangsstufe bestimmt.

Unter einer amorphen Struktur im Sinne dieser Erfindung wird verstanden, dass die Polyesterpolyole ihrer Struktur keine kristallinen Anteile ausbilden, so dass mittels DSC Messungen nur ein oder mehrere Glasübergangspunkte Tg, aber keine Schmelzpunkte oder Schmelzbereiche für die Polyesterpolyole gefunden werden können.

In einer besonders bevorzugten Ausführungsform der Erfindung umfasst die Komponente b) neben dem Polyesterpolyol b1) ein weiteres Polyesterpolyol b2).

Das Polyesterpolyol b2) enthält bevorzugt als Aufbaukomponente wenigstens eine aliphatische Dicarbonsäure und/oder das entsprechende Carbonsäureanhydrid. Als aliphatische Dicarbonsäure wird dabei bevorzugt Adipinsäure , gegebenenfalls in Mischung mit anderen aliphatischen Dicarbonsäuren und/oder Anhydriden eingesetzt..

Bevorzugt beträgt der Anteil an aliphatischen Dicarbonsäuren und/oder deren Anhydriden im Polyesterpolyol b2) ≥ 40 Gew.-%, besonders bevorzugt ≥ 60 Gew.-% und ganz besonders bevorzugt ≥ 70 Gew.-%, bezogen auf die Gesamtmasse des Polyesterpolyols b2).

Es werden bevorzugt ausschließlich aliphatische Dicarbonsäuren und/oder Anhydride und keine aromatischen Dicarbonsäuren und/oder Anhydride als Säurekomponente eingesetzt.

Bevorzugt enthält das Polyesterpolyol b2) als Diolkomponente verzweigte oder innerhalb der Hauptkette mit Heteroatomen substituierte Diole, insbesondere bevorzugt Butylenglykol, Diethylenglykol, Triethylenglykol, Polyalkylenglykole wie Polyethylenglykol, weiterhin 1,2-Propandiol und/oder Neopentylglykol und ganz besonders bevorzugt Diethylenglykol.

Bevorzugt ist das Polyesterpolyol b2) aufgebaut ausschließlich aus verzweigte oder innerhalb der Hauptkette mit Heteroatomen substituierte Diole, insbesondere bevorzugt Butylenglykol, Diethylenglykol, Triethylenglykol, Polyalkylenglykole wie Polyethylenglykol, weiterhin 1,2-Propandiol und/oder Neopentylglykol? und aliphatischen Dicarbonsäuren und/oder deren Anhydriden, besonders bevorzugt aus Diethylenglykol und Adipinsäure.

Bevorzugt weisen die eingesetzten Polyesterpolyole b2) eine amorphe Struktur auf.

Bevorzugt weisen die Polyesterpolyole b2) einen Glasübergangspunkt Tg, bestimmt mittels DSC, von ≤ - 10°C, besonders bevorzugt ≤ - 25°C und ganz besonders bevorzugt ≤ - 40°C auf.

Die Polyesterpolyole b1) und b2) liegen bevorzugt in einem Gewichtsverhältnis b1:b2 von 3:1 bis 1:4 und besonders bevorzugt von 2:1 bis 1:3 und insbesondere bevorzugt von 1:1 bis 1:2,5 vor. In einer besonders bevorzugten Ausführungsform liegt das Polyesterpolyol b2) im Überschuss vor.

Ganz besonders bevorzugt besteht die Komponente b) aus dem Polyesterpolyol b1) oder den Polyesterpolyolen b1) und b2), insbesondere bevorzugt aus den Polyesterpolyolen b1) und b2).

Insbesondere bevorzugt besteht die Komponente b) aus einem Polyesterpolyol b1), das als Säurekomponente ausschließlich aromatische Dicarbonsäuren und/oder die korrespondierenden Anhydride enthält und einem Polyesterpolyol b2), das als Säurekomponente aliphatische Dicarbonsäuren und/oder die korrespondierenden Anhydride enthält, wobei b1) eine amorphe Struktur aufweist.

Die Komponente c) umfasst wenigstens eine kationisch hydrophilierenden Komponente, die mindestens eine gegenüber Isocyanatgruppen reaktive Gruppe und mindestens eine kationische oder potentiell kationische Gruppe aufweist.

Bevorzugt weist die Komponente c) wenigstens eine tertiäre Aminogruppe und/oder eine Ammoniumgruppe auf.

Als Komponente c) geeignete Verbindungen sind beispielsweise Tris(hydroxyalkyl)amine, N, N'-bis(hydroxyalkyl)alkylamine, N-hydroxyalkyl-dialkylamine, Tris(aminoalkyl)amine, N, N'-bis(aminoalkyl)alkylamine, N-aminoalkyl-dialkylamine, sowie deren Gemische. Bevorzugt weisen die Alkylreste dabei 2 bis 6 Kohlenstoffatome auf.

Bevorzugt werden als Komponente c) N, N'-bis(hydroxyalkyl)alkylamine eingesetzt. Besonders bevorzugt sind diese ausgewählt aus N-Methyldiethanolamin, N-Ethyldiethanolamin, N-Propyldiethanolamin, N-Methyldipropanlamin, N-Ethyldiproopanolamin, N-Propyldipropanolamin und insbesondere bevorzugt ist die Komponente c) N-Methyldiethanolamin.

Die enthaltenen tertiären Aminogruppen können während oder nach der Herstellung des Po-lyurethanharnstoffs durch die Zugabe von Säuren teilweise oder komplett neutralisiert werden. Als Säuren werden dabei bevorzugt Phosphorsäure, Schwefelsäure, Halogensäuren und/oder organische Säuren wie Milchsäure, Ameisensäure und/oder Essigsäure, besonders bevorzugt organische Säuren und ganz besonders bevorzugt Essigsäure eingesetzt.

Der Anteil der Komponente c) am Polyurethanharnstoff ist bevorzugt ≥ 1 und ≤ 25 Gew.-%, besonders bevorzugt > 2 und ≤ 20 Gew.-% und ganz besonders bevorzugt > 5 und < 15 Gew.-%, bezogen auf das Gesamtgewicht des Polyurethanharnstoffs. Die Komponenten a) bis f) ergänzen sich dabei zu 100 Gew.-%.

Die Komponente d) umfasst wenigstens eine aliphatische, aminofunktionelle Verbindung, die mindestens zwei isocyanatreaktive Aminogruppen aufweist

Die Verbindungen der Komponente d) weisen bevorzugt keine hydrophilierenden Gruppen, insbesondere keine ionischen und/oder potentiell ionischen Gruppen auf.

Als Komponente d) geeignete Verbindungen sind insbesondere primäre und/oder sekundäre di-oder trifunktionale Amine, bevorzugt primäre und/oder sekundäre difunktionale Amine.

Da die Komponente d) zwei oder mehr isocyanatreaktive Aminogruppen aufweist, dient diese bevorzugt als Kettenverlängerer zum Aufbau höherer Molekulargewichte.

Geeignete Di- und Triamine sind beispielsweise 1,2-Ethandiamin, 1,6-Hexamethylendiamin, 1-amino-3,3,5-trimethyl-5-aminomethyl-cyclohexan (Isophorondiamin, IPDA), Piperazin, 1,4-diaminocyclohexan, bis-(4-aminocyclohexyl)-methan und Diethylenetriamine.

Bevorzugt werden als Komponente d) 1,2-Ethanediamin, 1-Amino-3,3,5-trimethyl-5-aminomethyl-cyclohexan (IPDA) und/oder Diethylentriamin eingesetzt.

Besonders bevorzugt umfasst die Komponente d) ≥ 90 Gew.-%, weiterhin bevorzugt ≥ 95 Gew.-% und insbesondere bevorzugt 100 Gew.-% IPDA, bezogen auf die Gesamtmasse der Komponente d).

Der Anteil der Komponente d) am Polyurethanharnstoff ist bevorzugt ≥ 0,5 und ≤ 20 Gew.-%, besonders bevorzugt ≥ 1 und ≤ 15 Gew.-% und ganz besonders bevorzugt ≥ 2 und ≤ 12 Gew.-%, bezogen auf das Gesamtgewicht des Polyurethanharnstoffs. Die Komponenten a) bis f) ergänzen sich dabei zu 100 Gew.-%.

Gegebenenfalls wird der Polyurethanharnstoff zudem aufgebaut aus Komponente e), ein oder mehrere Alkohole, die mindestens zwei Hydroxylgruppen und eine Molmasse von ≥ 60 und ≤ 399 g/mol aufweisen. Beispielsweise können die Polyole des genannten Molmassenbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit, sowie deren Gemische eingesetzt werden.

In einer bevorzugten Ausführungsform der Erfindung wird die Komponente e) eingesetzt.

Die Verbindungen der Komponente e) können prinzipiell nichtionisch hydrophilierende Gruppen aufweisen. Sie weisen jedoch bevorzugt keine ionisch oder nichtionisch hydrophilierenden Gruppen auf.

Der Anteil der Komponente e) am Polyurethanharnstoff ist bevorzugt ≥ 0 und ≤ 10 Gew.-%, besonders bevorzugt ≥ 0,5 und ≤ 6 Gew.-% und ganz besonders bevorzugt ≥ 1 und ≤ 4 Gew.-%, bezogen auf das Gesamtgewicht des Polyurethanharnstoffs. Die Komponenten a) bis f) ergänzen sich dabei zu 100 Gew.-%.

Weiterhin kann der Polyurethanharnstoff aufgebaut sein aus Komponente f), einer oder mehreren Verbindungen, die eine gegenüber Isocyanatgruppen reaktive Gruppe aufweisen, insbesondere Verbindungen die eine Amino- oder Hydroxygruppe aufweisen. Geeignete Verbindungen der Komponente f) sind beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Methanol, Ethanol, iso-Propanol, n-Propanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Die Verbindungen der Komponente f) können prinzipiell nichtionisch hydrophilierende Gruppen aufweisen. Sie weisen jedoch bevorzugt keine ionisch oder nichtionisch hydrophilierenden Gruppen auf.

Der Anteil der Komponente f) am Polyurethanharnstoff ist bevorzugt ≥ 0 und ≤ 10 Gew.-%, besonders bevorzugt ≥ 0 und ≤ 3 Gew.-% und ganz besonders bevorzugt 0, bezogen auf das Gesamtgewicht des Polyurethanharnstoffs. Die Komponenten a) bis f) ergänzen sich dabei zu 100 Gew.-%.

In einer bevorzugten Ausführungsform der Erfindung ist entweder die Komponente a) IPDI oder die Komponente d) IPDA oder die Komponente a) IPDI und die Komponente d) IPDA.

Weiterhin bevorzugt ist der Polyurethanharnstoff aufgebaut aus ≥ 10 und ≤ 65 Gew.-% der Komponente a), ≥ 5 und ≤ 70 Gew.-% der Komponente b), ≥ 2 und ≤ 20 Gew.-% der Komponente c), ≥ 1 und ≤ 15 Gew.-% der Komponente d), ≥ 0,5 und ≤ 6 Gew.-% der Komponente e) und ≥ 0 und ≤ 10 Gew.-% der Komponente f), jeweils bezogen auf die Gesamtmasse des Polyurethanharnstoffs, jeweils bezogen auf die Gesamtmasse des Polyurethanharnstoffs, wobei sich die Komponenten a) bis f) zu 100 Gew.-% ergänzen.

In einer bevorzugten Ausführungsform der Erfindung ist der erfindungsgemäß verwendete Po-lyurethanharnstoff aufgebaut aus
a) wenigstens einem aliphatischen, araliphatischen und/oder cycloaliphatischen Polyisocyanat, das ausgewählt ist aus HDI, H12-MDI und/oder IPDI,
b) einem oder mehreren Polyesterpolyolen mit einem zahlenmittleren Molekulargewicht Mn ≥ 400 und ≤ 6000 g/mol und einer Hydroxylfunktionalität von ≥ 1,5 und ≤ 4, wobei die Komponente b) wenigstens ein Polyesterpolyol b1) umfasst, das wenigstens > 45 Gew.-% einer aromatischen Dicarbonsäure und/oder des korrespondierende Säureanhydrids, bezogen auf die Gesamtmasse des Polyesterpolyols, als Aufbaukomponente enthält,
c) wenigstens einer kationisch hydrophilierenden Komponente, die mindestens eine gegenüber Isocyanatgruppen reaktive Gruppe und mindestens eine tertiäre Aminogruppe und/oder eine Ammoniumgruppe aufweist,
d) wenigstens einem aliphatischen primären oder sekundären Diamin, das zwei isocyanatreaktive Aminogruppen und keine ionischen und/oder potentiell ionischen Gruppen aufweist,
e) wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist.

Weiterhin bevorzugt ist der Polyurethanharnstoff dieser Ausführungsform aufgebaut aus ≥ 10 und ≤ 65 Gew.-% der Komponente a), ≥ 5 und ≤ 70 Gew.-% der Komponente b), ≥ 2 und ≤ 20 Gew.-% der Komponente c), ≥ 1 und ≤ 15 Gew.-% der Komponente d), ≥ 0,5 und ≤ 6 Gew.-% der Komponente e) und ≥ 0 und ≤ 10 Gew.-% der Komponente f), jeweils bezogen auf die Gesamtmasse des Polyurethanharnstoffs, jeweils bezogen auf die Gesamtmasse des Polyurethanharnstoffs, wobei sich die Komponenten a) bis f) zu 100 Gew.-% ergänzen.

In einer besonders bevorzugten Ausführungsform der Erfindung ist der erfindungsgemäß verwendete Polyurethanharnstoff ausschließlich aufgebaut aus
a) wenigstens einem aliphatischen, araliphatischen und/oder cycloaliphatischen Polyisocyanat, wobei die Komponente a) ≥ 95 Gew.-% an IPDI umfasst,
b) Polyesterpolyol b1), das als Säurekomponente ausschließlich aromatische Dicarbonsäuren oder die korrespondierenden Anhydride enthält und Polyesterpolyol b2), das als Säurekomponente ausschließlich aliphatische Dicarbonsäuren oder die korrespondierenden Anhydride enthält,
c) wenigstens einer kationisch hydrophilierenden Komponente, die ausgewählt ist aus N, N'-bis(hydroxyalkyl)alkylaminen,
d) wenigstens einer aliphatischen, aminofunktionellen Verbindung, die mindestens zwei isocyanatreaktive Aminogruppen aufweist, wobei die Komponente a) ≥ 95 Gew.-% an IPDA umfasst,
e) wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist und keine ionisch oder nichtionisch hydrophilierenden Gruppen enthält.

Weiterhin bevorzugt ist der Polyurethanharnstoff der vorstehenden Ausführungsform aufgebaut aus ≥ 10 und ≤ 65 Gew.-% der Komponente a), ≥ 5 und ≤ 70 Gew.-% der Komponente b), ≥ 2 und ≤ 20 Gew.-% der Komponente c), ≥ 1 und ≤ 15 Gew.-% der Komponente d), ≥ 0,5 und ≤ 6 Gew.-% der Komponente e) und ≥ 0 und ≤ 10 Gew.-% der Komponente f), jeweils bezogen auf die Gesamtmasse des Polyurethanharnstoffs, jeweils bezogen auf die Gesamtmasse des Polyurethanharn-stoffs, wobei sich die Komponenten a) bis f) zu 100 Gew.-% ergänzen.

Vorteilhaft weist der Polyurethanharnstoff ein zahlenmittleres Molekulargewicht Mn ≥ 3000 und ≤ 50000 g/mol, besonders vorteilhaft ≥ 5000 und ≤ 30000 g/mol, auf.

Für die Herstellung der Polyurethanharnstoffe werden bevorzugt die Komponenten a), b) und c) sowie gegebenenfalls e) und f) zur Herstellung eines NCO-terminierten Präpolymers ganz oder teilweise vorgelegt, gegebenenfalls mit einem gegenüber Isocyanatgruppen inerten Lösungsmittel verdünnt und auf Temperaturen im Bereich von 50 bis 120°C aufgeheizt. Die Herstellung der Präpolymere erfolgt dabei bevorzugt in einem Schritt, kann aber auch stufenweise erfolgen.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, bevorzugt werden solche Lösemittel eingesetzt, die mit Wasser mischbar sind..

Zur Beschleunigung der Isocyanatadditionsreaktion können die in der Polyurethan-Chemie bekannten Katalysatoren eingesetzt werden. In einer bevorzugten Variante wird jedoch ohne den Zusatz von Urethanisierungs-Katalysatoren gearbeitet.

Bei der Herstellung der NCO-terminierten Präpolymere aus den Komponenten a), b) und c) sowie gegebenenfalls e) und f) beträgt das Stoffmengenverhältnis von Isocyanat-Gruppen zu isocyanatreaktiven Gruppen im allgemeinen ≥ 1,05 und ≤ 2,5, bevorzugt ≥ 1,15 und ≤ 1,95, besonders bevorzugt ≥ 1,2 und ≤ 1,7.

In einem sich anschließenden Schritt wird bevorzugt dann das im ersten Schritt erhaltene NCO-terminierte Präpolymer ganz oder teilweise mit der Komponente d) sowie gegebenenfalls den Komponenten c), e) und f) umgesetzt. Bevorzugt wird die Komponente c) nicht eingesetzt, besonders bevorzugt erfolgt die Umsetzung nur mit der Komponente d). Diese Umsetzung wird im allgemeinen als Kettenverlängerung, bzw. im Fall der Komponente f) als Kettenabbruch bezeichnet. Die Umsetzung kann in einem Schritt oder stufenweise erfolgen.

Bevorzugt wird dabei das NCO-terminierte Präpolymer vorgelegt und die Komponenten d) sowie gegebenenfalls c), e) und f) zudosiert. Die Komponenten d) und gegebenenfalls c), e) und f) können dabei auch stufenweise in mehreren Schritten, insbesondere in zwei Schritten, zugegeben werden. Die Komponenten d) sowie gegebenenfalls c), e) und f) können in Wasser oder organischen Lösemitteln eingesetzt werden.

Die Zugabe der Komponenten d) sowie gegebenenfalls c), e) und f) erfolgt bevorzugt bei Temperaturen von 10 bis 100°C, vorzugsweise 25 bis 60 °C.

Der Kettenverlängerungsgrad, also das molare Verhältnis von NCO-reaktiven Gruppen der zur Kettenverlängerung und Kettenabbruch eingesetzten Komponenten d) sowie gegebenenfalls c), e) und f) zu freien NCO-Gruppen des Präpolymers, liegt im allgemeinen > 25 und ≤ 150%, bevorzugt ≥ 50 und ≤ 120%, besonders bevorzugt > 40 und ≤ 100%.

Enthält die Komponente c) potentiell kationische Gruppen, so können diese durch Neutralisation, bevorzugt mit einer der oben genannten Säuren, ganz oder teilweise in kationische Gruppen überführt werden.

Die Stoffmenge der Säure beträgt bevorzugt zwischen 50 und 125 mol%, besonders bevorzugt zwischen 70 und 100 mol%, der Stoffmenge der zu neutralisierenden Gruppen. Die Neutralisation kann vor, während oder nach der Umsetzung des NCO-terminierten Präpolymers erfolgen.

Der Polyurethanharnstoff ist bevorzugt erhältlich durch Umsetzung der Komponenten a), b), c) und gegebenenfalls e) und f) zu einem Isocyanat-terminierten Präpolymer, anschließender Umsetzung des Präpolymers mit der Komponente d) und gegebenenfalls den Komponenten e) und f) und falls die Komponente c) eine potentiell kationische Gruppe umfasst Neutralisation des Polyurethanharn-stoffs durch eine Säure vor, während oder nach der Umsetzung des Isocyanat-terminierten Präpolymers mit der Komponente d) und gegebenenfalls den Komponenten e) und f).

Die Dispergierung des Polyurethanharnstoffs in Wasser kann vor, während oder nach der Umsetzung des NCO-terminierten Präpolymers erfolgen. Die Dispergierung kann während oder nach der Neutralisation erfolgen. Die Neutralisation kann auch gleichzeitig mit der Dispergierung erfolgen, in dem das Dispergierwasser bereits das Neutralisationsmittel enthält.

Die Dispergierung erfolgt bevorzugt im Anschluss an die Umsetzung des-NCO terminierten Präpolymers. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben. Zur Herstellung der Polyurethandispersion können alle aus dem Stand der Technik bekannten Verfahren wie z. B. Prepolymer-Mischverfahren, Acetonverfahren oder Schmelzdispergierverfahren verwendet werden. Bevorzugt wird das Aceton-Verfahren angewandt.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den so hergestellten Polyurethan-Dispersionen beträgt bevorzugt 0 bis 10 Gew.-%, besonders bevorzugt 0 bis 3 Gew.-%, bezogen auf die gesamte Dispersion.

Der Feststoffanteil des Polyurethanharnstoffs an der erfindungsgemäß eingesetzten Polyurethan-harnstoffdispersion liegt dabei bevorzugt ≥ 10 und ≤ 80 Gew.-%, besonders bevorzugt ≥ 15 und ≤ 60 Gew.-% und ganz besonders bevorzugt bei ≥ 20 und ≤ 50 Gew.-%, bezogen auf das Gesamtgewicht der Polyurethanharnstoffdispersion.

Die Polyurethanharnstoffdispersion weist bevorzugt eine Viskosität ≥ 10 und ≤ 10000 mPas, besonders bevorzugt Viskosität ≥ 50 und ≤ 15000 mPas, bestimmt mittels Rotationsviskosimetrie nach DIN 53019 bei 23°C, auf.

Die erfindungsgemäße Nagellackzusammensetzung enthält bevorzugt ≥ 0,1 und ≤ 60 Gew.-% des oben beschriebenen kationischen Polyurethanharnstoffs (bezogen auf den Aktivstoff selbst und nicht die wässrige Dispersion), insbesondere bevorzugt ≥ 1 und ≤ 50 Gew.-% und ganz besonders bevorzugt > 2 und ≤ 40 Gew.-%, jeweils bezogen auf das Gesamtgewicht der Zusammensetzung.

Die erfindungsgemäße Nagellackzusammensetzung enthält bevorzugt > 2 und ≤ 99 Gew.-% der wässrigen Polyurethanharnstoffdispersion, insbesondere bevorzugt ≥ 10 und ≤ 97 Gew.-% und ganz besonders bevorzugt ≥ 20 und ≤ 95 Gew.-%, jeweils bezogen auf das Gesamtgewicht der Zusammensetzung.

Der Anteil an organischem Lösemittel, bezogen auf die gesamte Nagellackzusammensetzung, beträgt bevorzugt ≤ 5 Gew.-%, besonders bevorzugt ≤ 1 Gew.-% und ganz besonders bevorzugt 0 Gew.-%.Unter organischen Lösemitteln im Sinne der Erfindung werden insbesondere organische Substanzen verstanden, die bei Raumtemperatur und bei 101,325 kPa flüssig sind, eine Viskosität von ≤ 100 mPas bei 23 °C und einen Siedepunkt von ≤ 250 °C bei 101,325 kPa aufweisen. Wasser ist kein organisches Lösemittel im Sinne der Erfindung.

Organische Lösemittel im Sinne der Erfindung sind insbesondere
- Ketone, wie zum Beispiel Methylethylketon, Methylisobutylketon, Diisobutylketon, Isophoron, Cyclohexanon, Aceton;
- aliphatische Monoalkohole mit ein bis fünf Kohlenstoffatomen, die bei Raumtemperatur flüssig sind, wie zum Beispiel Ethanol, Butanol, Propanol, Isopropanol, Diacetonalkohol, 2-Butoxyethanol, Cyclohexanol;
- Polyole, insbesondere Glykole, wie zum Beispiel Ethylenglykol, Propylenglykol, Pentylenglykol, Glycerol, Dipropylenglykol, Butylen-1,3-glykol, Polypropylenglykol;
- Glykolether wie Alkyl (C1-4)ether von Mono-, Di- oder Tripropylenglykol oder Mono-; Di- oder Triethylenglykol, die bei Raumtemperatur flüssig sind, wie zum Beispiel Propylenglykolmonomethylether, Propylenglykolmonomethyletheracetat, Dipropylenglykolmono-n-butylether;
- Cyclische Ether, wie zum Beispiel γ-Butyrolacton;
- Kurzkettige Ester mit drei bis acht Kohlenstoffatomen, wie zum Beispiel Ethylacetat, Methylacetat, Propylacetat, Isopropylacetat, n-Butylacetat, Isopentylacetat, Methoxypropylacetat, t-Butylacetat, Butylacetat;
- Ether, die bei Raumtemperatur flüssig sind, wie zum Beispiel Diethylether, , *tert.*-Butylmethylether
- Alkane, die bei Raumtemperatur flüssig sind, wie zum Beispiel Hexan, Heptan, Decan, , Dodecan, Cyclohexan;
- Alkylsulfoxide, wie zum Beispiel Dimethylsulfoxid;
- Aldehyde mit zwei bis vier Kohlenstoffatomen, die bei Raumtemperatur flüssig sind, wie zum Beispiel Benzaldehyd, Acetaldehyd;
- Carbonate, wie zum Beispiel Propylencarbonat, Dimethylcarbonat;
- Acetale, wie zum Beispiel Methylal;
und Gemische der genannten organischen Lösungsmittel.

Der Wasseranteil der erfindungsgemäßen Nagellackzusammensetzung liegt bevorzugt im Bereich von ≥ 10 und ≤ 95 Gew.-%, bevorzugt im Bereich von ≥ 40 und ≤ 90 Gew.-%, ganz bevorzugt im Bereich von ≥ 60 und ≤ 85 Gew.-%, bezogen auf das Gesamtgewicht der Zusammensetzung, wobei das Wasser dabei aus der wässrigen Dispersion stammt und/oder zusätzlich zugesetzt wird.

Das verwendete Wasser in der erfindungsgemäßen Nagellackzusammensetzung kann reines demineralisiertes Wasser, Leitungswasser, Mineralwasser, Thermalwasser und/oder Meerwasser sein, vorzugsweise wird demineralisiertes Wasser eingesetzt.

Bevorzugt enthalten die erfindungsgemäßen Nagellackzusammensetzungen keine Polyalkylenamine. Weiterhin bevorzugt enthalten die erfindungsgemäßen Nagellackzusammensetzungen keine Alkoxysilane, die eine solubilisierende Gruppe im Sinne von WO2012/061265 aufweisen.

Die erfindungsgemäße Nagellackzusammensetzung ist bevorzugt frei von Nitrocellulose und deren Derivaten.

Die erfindungsgemäße Zusammensetzung kann neben dem oben beschriebenen Polyurethanharn-stoff weitere geeignete Filmbildner enthalten.

Die Konzentration eines oder mehrerer weiterer Filmbildner kann von ≥ 0 und ≤ 40 Gew.-% und insbesondere ≥ 0,1 und ≤ 30 Gew.-% jeweils bezogen auf das Gesamtgewicht der Zusammensetzung betragen.

Vorteilhaft werden der oder die Filmbildner gewählt aus der Gruppe der von den erfindungsgemäß verwendeten Polyurethanharnstoffen verschiedenen wasserlöslichen oder wasserdispergierbaren Polyurethane, der Polyharnstoffe, Silikonharze und/oder Polyester sowie der nichtionischen, anionischen, amphoteren und/oder kationischen Polymere und ihren Mischungen.

Vorteilhafte nichtionische Polymere, die in erfindungsgemäßen Nagellackzusammensetzungen alleine oder im Gemisch, vorzugsweise auch mit anionischen und/oder amphoteren und/oder zwitterionischen Polymeren enthalten sein können, sind ausgewählt aus:
- Polyalkyloxazoline,
- Vinylacetat Homo- oder Copolymerisate. Hierzu gehören beispielweise Copolymerisate aus Vinylacetat und Acrylsäureester, Copolymerisate aus Vinylacetat und Ethylen, Copolymerisate aus Vinylacetat und Maleinsäureester,
- Acrylsäureester Copolymerisate wie z. B. die Copolymerisate aus Alkyl-Acrylat und Alkyl-Methacrylat, Copolymerisate aus Alkyl-Acrylat und Urethanen,
- Copolymerisate aus Acrylnitril und nichtionischem monomer ausgewählt aus Butadien und (Meth)Acrylat,
- Styrol Homo- und Copolymerisate. Hierzu gehören beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin,
- Polyamide,
- Vinyllactame Homo- oder Copolymere, wie Vinylpyrrolidon-Homo- oder Copolymerisate;
   hierzu gehören beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat,
- Polysiloxane,
- Homopolymere des N-Vinylformamids z. B. PVF von Akzo Nobel.
- Besondere bevorzugte nichtionische Polymere sind Acrylsäureester Copolymerisate, Homopolymere des Vinylpyrrolidons und Copolymere, Polyvinylcaprolactam.
- Ganz besondere bevorzugte nichtionische Polymere sind Homopolymere des Vinylpyrrolidons z. B. Luviskol® K von der Firma BASF, Copolymerisate aus Vinylpyrrolidon und Vinylacetat z. B. Luviskol® VA Typen der Firma BASF oder PVPVA® S630L der Firma Ashland, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte anionische Polymere sind Homo-oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen, insbesondere Carbonsäure, Sulfonsäure oder Phosphonsäure.

Vorteilhafte anionische Polymere enthaltend Carbonsäuregruppe sind:
- Acrylsäure oder Methacrylsäure Homo- oder Copolymer oder die Salze davon. Hierzu gehören beispielweise die Copolymerisate aus Acrylsäure und Acrylamide und/oder deren Natriumsalze, Copolymerisate aus Acrylsäure und/oder Methacrylsäure und einem ungesättigten Monomer ausgewählt aus Ethylene, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone® LM der Firma Ashland, Copolymerisate aus Methacrylsäure, Ethylacrylate und Tert-butyl Acrylate z.B. Luvimer® 100 P der Firma BASF.
- Crotonsäurederivat-Homo- oder Copolymer oder die Salze davon. Hierzu gehören beispielweise Vinylacetat/Crotonsäure-, Vinylacetat/Acrylat- und/oder Vi-nylacetat/Vinylneodecanoat/Crotonsäure-Copolymere, Natriumacrylat/Vinylalkohol-Copolymere,
- ungesättigte C4-C8 Carbonsäurederivate oder Carbonsäureanhydrid Copolymer ausgewählt aus Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Allylester, Methallylester und ggfs. Acrylamide, Methacrylamide, alpha-Olefin, Acrylsäureester, Methacrylsäureester, Vinylpyrrolidone. Weitere bevorzugte Polymere sind Methylvinylether/MaleinsäureCopolymere, die durch Hydrolyse von Vinylether/Maleinsäureanhydrid-Copolymeren entstehen. Diese Polymere können auch teilverestert (Ethyl, Isopropyl- bzw. Butylester) oder teilamidiert sein.
- in Wasser loesliche oder dispergierbare anionische Polyurethane, z. B. Luviset ®PUR von BASF, die von den erfindungsgemäßen Polyurethanen verschieden sind,
wobei diese Aufstellung selbstverständlich nicht limitierend sein soll.

Vorteilhafte anionische Polymere enthaltend Sulfonsäuregruppe sind Salze von Polyvinylsulfonsäure, Salze von Polystyrolsulfonsäure wie z. B. Natriumpolystyrolsulfonat oder Salze von Polyacrylamidesulfonsäure.

Vorteilhafte amphotere Polymere können unter den Polymeren ausgewählt werden, die statistisch in der Polymerkette verteilte Einheiten A und B enthalten, wobei A eine Einheit bedeutet, die von einem Monomer mit mindestens einem basischen Stickstoffatom abgeleitet ist, und B eine Einheit ist, die von einem sauren Monomer stammt, das eine oder mehrere Carboxygruppen oder Sulfonsäuregruppen aufweist, oder A und B können Gruppen bedeuten, die von zwitterionischen Carboxybetainmonomeren oder Sulfobetainmonomeren abgeleitet sind; A und B können auch eine kationische Polymerkette bedeuten, die primäre, sekundäre, tertiäre oder quartanäre Gruppen enthält, worin mindestens eine Aminogruppe eine Carboxygruppe oder Sulfonsäuregruppe trägt, die über eine Kohlenwasserstoffgruppe gebunden ist, oder B und C sind Teil einer Polymerkette mit Ethylen-α,β-dicarbonsäureeinheit, bei der die Carbonsäuregruppen mit einem Polyamin umgesetzt wurden, das eine oder mehrere primäre oder sekundäre Aminogruppen enthält.

### Besondere vorteilhafte amphotere Polymere sind:

- Polymere, die bei der Copolymerisation eines von einer Vinylverbindung mit Carboxygruppe abgeleiteten Monomers, wie insbesondere Acrylsäure, Methacrylsäure, Maleinsäure, α-Chloracrylsäure, und einem basischen Monomer gebildet werden, das von einer Vinylverbindung abgeleitet ist, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent Nr. 3 836 537 beschrieben worden.
- Polymere mit Einheiten, die abgeleitet sind von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat.

Erfindungsgemäß besonders bevorzugte N-substituierte Acrylamide oder Methacrylamide sind Verbindungen, deren Alkylgruppen 2 bis 12 Kohlenstoffatome enthalten, besonders N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.

Die sauren Comonomere sind insbesondere unter Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.

Bevorzugte basische Comonomere sind Aminoethylmethacrylat, Butylaminoethylmethacrylat, N,N-Dimethylaminoethylmethacrylat, N-t-Butylaminoethylmethacrylat.
- Vernetzte und ganz oder teilweise acylierte Polyaminoamide, die von Polyaminoamiden der folgenden allgemeinen Formel abgeleitet sind:

   -[CO-R-CO-Z]-

   worin R eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bissekundäres Amin entsteht, und Z eine Gruppe bedeutet, die von einem bis-primären, mono-oder bis-sekundären Polyalkylenpolyamin abgeleitet ist, und vorzugsweise: a) in Mengenanteilen von 60 bis 100 Mol-% die Gruppen -NH-[(CH₂)ₓ-NH-]ₚ- mit x = 2 und p = 2 oder 3 oder x = 3 und p = 2, wobei diese Gruppe, von Diethylentriamin, Triethylentetramin oder Dipropylentriamin abgeleitet ist; b) in Mengenanteilen von 0 bis 40 Mol-% die Gruppe-NH-[(CH₂)ₓ-NH-]ₚ-, worin x = 2 und p = 1, die von Ethylendiamin abgeleitet ist, oder die Gruppe, die von Piperazin stammt: 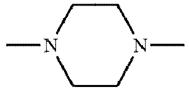 c) in Mengenanteilen von 0 bis 20 Mol-%, die Gruppe -H-(CH₂)₆-NH-, die von Hexamethylendiamin abgeleitet ist, wobei diese Polyaminoamide durch Addition eines bifunktionellen Vernetzungsmittels, das unter den Epihalohydrinen, Diepoxiden, Dianhydriden und bis-ungesättigten Derivaten ausgewählt ist, in einer Menge von 0,025 bis 0,35 mol Vernetzungsmittel pro Aminogruppe des Polyaminoamids vernetzt und mit Acrylsäure, Chloressigsäure oder einem Alkansulfon oder deren Salzen acyliert ist.

Die gesättigten Carbonsäuren sind vorzugsweise unter den Säuren mit 6 bis 10 Kohlenstoffatomen ausgewählt, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure, Methacrylsäure und Itaconsäure.

Die bei der Acylierung verwendeten Alkansulfone sind vorzugsweise Propansulfon oder Butansulfon, die Salze der Acylierungsmittel sind vorzugsweise die Natriumsalze oder Kaliumsalze.
- Polymeren mit zwitterionischen Einheiten der folgenden Formel: 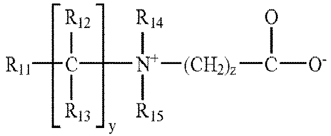 worin R₁₁ eine polymerisierbare ungesättigte Gruppe bedeutet, wie Acrylat, Methacrylat, Acrylamid oder Methacrylamid, y und z ganze Zahlen von 1 bis 3 bedeuten, R₁₂ und R₁₃ ein Wasserstoffatom, Methyl, Ethyl oder Propyl bedeuten, R₁₄ und R₁₅ ein Wasserstoff-atom oder eine Alkylgruppe bedeuten, die so gewählt ist, dass die Summe der Kohlenstoff-atome R₁₄ und R₁₅ 10 nicht übersteigt.
   Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 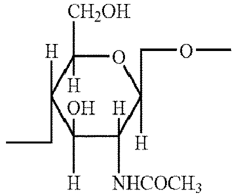 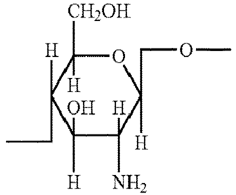 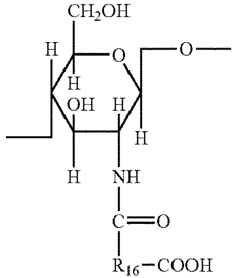 wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 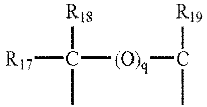 worin bedeuten: falls q = 0, die Gruppen R₁₇, R₁₈ und R₁₉, die gleich oder verschieden sind, jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoff-atome unterbrochen und/oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet; oder falls q = 1, die Gruppen R₁₇, R₁₈ und R₁₉ jeweils ein Wasserstoff-atom, sowie die Salze, die diese Verbindungen mit Basen oder Säuren bilden.
- Polymere, die der folgenden allgemeinen Formel entsprechen und die beispielsweise in dem französischen Patent 1 400 366 beschrieben sind: 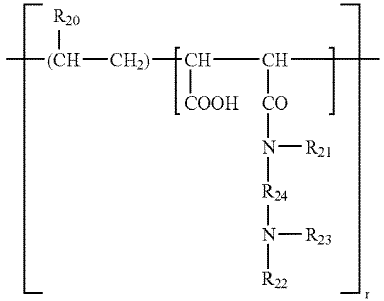 worin R₂₀ ein Wasserstoffatom, CH₃O, CH₃CH₂O oder Phenyl bedeutet, R₂₁ ein Wasserstoffatom oder eine niedere Alkylgruppe, wie Methyl oder Ethyl ist, R₂₂ ein Wasserstoff-atom oder eine niedere C₁₋₆-Alkylgruppe bedeutet, wie Methyl oder Ethyl, R₂₃ eine niedere C₁₋₆-Alkylgruppe ist, wie Methyl oder Ethyl oder eine Gruppe der Formel: -R₂₄-N(R₂₂)₂,
   wobei R₂₄ eine Gruppe -CH₂-CH₂, -CH₂-CH₂-CH₂- oder -CH₂-CH(CH₃)- bedeutet und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.
- Alkyl(C1-5)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Gegebenenfalls einsetzbar sind kationische Polymere, wie beispielweise Polymere, die primäre, sekundäre tertiäre und/oder quaternäre Aminogruppen enthalten, die Teil der Polymerkette oder direkt an die Polymerkette gebunden sind.

Ferner können die erfindungsgemäßen Nagellacke auch Additive wie Farbstoffe, Pigmente, Antioxidantien, Lichtschutzmittel, Benetzungsmittel, Emulgatoren, Dispergiermittel, Stabilizator, Entschäumer, Füllstoffe, Verlaufsmittel, Koalenszensmittel, Weichmacher, Konservierungsmittel, feuchtigkeitsspendende Substanzen, Parfum, Radikalfänger, Verdickungsmittel, Neutralizationsmittel, Öle, Wachse, Füllstoffe und Wirkstoffe enthalten sein. Je nach gewünschtem Eigenschaftsbild und Verwendungszweck der erfindungsgemäßen Nagellacke können bis zu 90 Gew.-%, bezogen auf Gesamttrockensubstanz, dieser Additive im Endprodukt enthalten sein.

Um die Filmbildungseigenschaften der erfindungsgemäßen Nagellacke zu verbessern können Filmbildungshilfemittel eingesetzt werden. Das Filmbildungshilfemittel kann aus der Gruppe von den Weichmachern und/oder Koalenszensmitteln ausgewählt werden.

Die bevorzugten Weichmacher sind beispielweise Adipate wie beispielweise Diethyladipat, Dibutyladipat, Diisobutyladipat, Diisopropyladipat, Sebacate wie beispielweise Dimethylsebacat, Diethylsebacat, Dibutylsebacat, Citrate wie beispielweise Triethylacetylcitrat, Tributylacetylcitrat Phtalate wie beispielweise Diethylphthalat, Dibutylphthalat, Dioctylphthalat und Gemischen davon.

Der Mengenanteil an Weichmacher in der erfindungsgemäßen Zusammensetzung kann beispielsweise im Bereich von ≥ 0 und ≤ 20 Gew.-% und bevorzugt ≥ 0,1 und ≤ 10 Gew.-%, bezogen auf das Gesamtgewicht der Zusammensetzung, liegen.

Die bevorzugten Koalenszensmitteln sind beispielweise Propylenglykol Ether wie zum Beispiel Propylenglykol-n-butylether, Propylenglykol-t-butylether, Propylenglykol n-Propylether, Propylenglykol Phenylether, Dipropylenglykol Ether wie zum Beispiel Dipropylenglycol-n-butylether, Dipropylenglykol-methylether, Dipropylenglykol-t-butylether, Dipropylenglycol-n-propylether, Propylenglykolmethylether Acetat, Propylenglykoldiacetat, Methyllactat, Ethyllactat, Isopropy-llactat, Butyllactate und Gemischen davon.

Der Mengenanteil an Koalenszensmitteln in der erfindungsgemäßen Zusammensetzung kann beispielsweise im Bereich von ≥ 0 und ≤ 10 Gew.-% und bevorzugt ≥ 0,1 und ≤ 8 Gew.-%, bezogen auf das Gesamtgewicht der Zusammensetzung, liegen.

Die erfindungsgemäße Zusammensetzung kann einen effektgebenden Bestandteil enthalten. Der genannte Bestandteil kann insbesondere farbgebend sein aber auch sonstige andere Effekte bereitstellen, wie Glitzer- und/oder Metalliceffekte. Bevorzugt enthält die erfindungsgemäße Zusammensetzung mindestens einen Farbstoff, der bevorzugt aus der Gruppe von lipophilen Farbstoffen, hydrophilen Farbstoffen, Pigmenten, Pailletten und Perlmutt ausgewählt wird. Erfindungsgemäß besonders vorteilhaft ist die Konzentration von Farbstoffen ≥ 0 und ≤ 50 Gew.-%, besonders vorteilhaft ≥ 0,1 und ≤ 30 Gew.-% Gewichts-%, ganz besonders vorteilhaft von ≥ 0,5 und ≤ 15 Gew.-%, jeweils bezogen auf das Gesamtgewicht der Zusammensetzung.

Beispielweise können lipophile Farbstoffe verwendet werden, wie Sudan I (gelb), Sudan II (orange), Sudan III (rot), Sudan IV (Scharlachrot), DC Red 17, DC Green 6, β-carotin, Sojaöl, DC Yellow 11, DC Violet 2, DC Orange 5 und DC Yellow 10.

Beispielweise können hydrophile Farbstoffe verwendet werden, wie Rüben-Saft und Methylenblau. Die Pigmente können prinzipiell alle anorganischen oder organischen Pigmente sein, die in kosmetischen oder dermatologischen Zusammensetzung verwendet werden. Die erfindungsgemäßen verwendeten Pigmente können beispielsweise weiß oder farbig sein, sie können umhüllt bzw. beschichtet sein mit einem hydrophoben Behandlungsmittel oder nicht beschichtet sein.

Vorteilhaft werden die Pigmente gewählt aus der Gruppe der Metalloxide, wie die Oxide von Eisen (insbesondere die Oxide von gelber, roter, brauner, schwarzer Farbe), Titandioxid, Zinkoxid, Ceroxid, Zirconiumoxid, Chromoxid; Manganviolett, Ultramarinblau, Preussisch Blau, Ultramarin und Eisenblau, Bismutoxidchlorid, Perlmutt, mit Titan oder Bismutoxidchlorid überzogene Glimmer-Pigmente, farbige Perlglanzpigmente, beispielsweise Titan-Glimmer-Pigmente mit Eisenoxiden, Titan-Glimmer-Pigmente insbesondere mit Eisenblau oder Chromoxid, Titan-Glimmer-Pigmente mit einem organischen Pigment vom vorgenannten Typ, sowie Perlglanzpigmente auf der Basis von Bismutoxidchlorid, Russ, die Pigmente vom Typ D & C, wie D&C Red N° 5, 6, 7, 10, 11, 12, 13, 34, D&C Yellow Lake N°5 und D&C Red Lake N° 2 und die Lacke auf der Basis von Cochenillerot, Barium, Strontium, Calcium und Aluminium und deren Gemische.

Zur Einstellung rheologischer Eigenschaften können Verdickungsmittel als Additive eingesetzt werden. Erfindungsgemäß besonders vorteilhaft ist die Konzentration von Verdickungsmittel ≥ 0 und ≤ 20 Gew.-%, besonders vorteilhaft ≥ 0,1 und ≤ 15 Gew.-%, ganz besonders vorteilhaft von ≥ 0,5 und ≤ 10 Gew.-%, jeweils bezogen auf das Gesamtgewicht der Zusammensetzung.

Vorteilhafte Verdickungsmittel sind:
- Vernetzte oder nichtvernetzte Acrylsäure- oder Methacrylsäure-Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate und Vinylacetat.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure angegeben werden.
- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan, organomodifizierte Tone wie organomodifizierte Bentonite, Stearalkonium Bentonite, organomodifizierte Hectorite, Stearalkonium Hectorite, oder organomodifizierte Montmorillonit, hydrophobe pyrogene Kieselsäure, wobei die Silanolgruppen mit Trimethylsiloxygruppen substituiert sind (AEROSIL® R812 der Firma Evonik) oder mit Dimethylsiloxygruppen oder Polydimethylsiloxan (AEROSIL®) R972, AEROSIL®) R974 von Evonik, CAB-O-SIL® TS-530, CAB-O-SIL® TS-610, "CAB-O-SIL® TS-720 von Cabot), Polysaccharide Alkylether (wie in EP 898958-A beschrieben).
- Nichtionische, anionische, kationische oder amphotere assoziative Polymere z.B. auf Basis von Polyethylenglycole und ihre Derivate, oder Polyurethane.

Besondere vorteilhafte Verdickungsmittel sind organomodifizierte Tone basiert auf Bentonite, Hectorite und Laponite, assoziative Polymere, Cellulose und ihre Derivative wie beispielweise Hydroxyethylcellulose und Carboxymethylcellulose.

Die erfindungsgemäßen Nagellacke können Füllstoffe enthalten. Unter Füllstoffe sind farblose oder weiße, mineralische oder synthetische, unlöslich in dem Medium der erfindungsgemäßen Nagellacke, lamellare, sphärische oder längliche inerte Partikel Füllstoffe zu verstehen, welche z.B. die rheologischen Eigenschaften und die Textur der Formulierungen modifizieren.

Die Füllstoffe können in der erfindungsgemäßen Zusammensetzung beispielsweise in einer Menge von ≥ 0 und ≤ 50 Gew.-%, bezogen auf das Gesamtgewicht der Zusammensetzung, und vorzugsweise von ≥ 0,1 und ≤ 30 Gew.-% enthalten sein.

Vorteilhafte partikuläre Füllstoffe im Sinne der vorliegenden Erfindung sind Talkum, Glimmer (Mica), Siliciumdioxid, Kaolin, Stärke und deren Derivaten (beispielweise Tapiocastärke, Distärkephosphat, Aluminium- bzw. Natrium-Stärke Octenylsuccinat und dergleichen), pyrogene Kieselsäure, Pigmente, die weder hauptsächlich UV-Filter-noch färbende Wirkung haben (wie z.B. Bornitrid etc.), Bornitrid, Calciumcarbonat, Dicalciumphosphat, Magnesiumcarbonat, Magnesiumhydrogencarbonat, Hydroxyapatite, mikrokristalline Cellulose, Pulver von synthetischen Polymeren, wie Polyamide (beispielsweise die unter der Handelsbezeichnung "Nylon®)" erhältlichen Polymeren), Polyethylen, Poly-β-alanin, Polytetrafluorethylen ("Teflon®"), Polyacrylat, Polyurethan, Lauroyl-lysine, Silikonharz (beispielsweise die unter der Handelsbezeichnung "Tospearl®" der Firma Momentive Performance Materials. erhältlichen Polymeren), Polyorganosiloxanelastomer, Hohlpartikel von Polyvinyliden/Acrylonitrile (Expancel® der Firma Akzo Nobel) oder Hohlpartikel von Siliciumoxid (Silica Beads® der Firma MAPRECOS), Glas-oder Keramik-Mikrokapseln, und Metallseifen, die von organischen Carbonsäuren mit 8 bis 22 Kohlenstoffatomen, vorzugsweise 12 bis 18 C-Atomen abgeleitet werden, wie zum Beispiel Zinkstearat, Magnesiumstearat oder Lithiumstearat, Zinklaurat, Magnesiummyristat.

In besonderem Vorteil der erfindungsgemäßen Nagellacke besteht darin, dass in den Lack auch die Fingernägel pflegende Zusätze eingearbeitet werden können. Geeignet sind beispielsweise Vitamine B5, E und C und deren Derivativen sowie Dimethyloxobenzodioxasilane, Calciumchlorid, Calciumpantothenat, Panthenol, Proteine, Ceramide, Myrrhe, Pflanzenextrakte, Aminosäure Öle wie zum Beispiel Cystein und deren Salzen und Derivaten, Cystein, Glutathion, Biotin, Harnstoff und Dimethylharnstoff., alpha-Hydroxysäuren, wie Citronensäure und Ascorbinsäure, UV-Schutzmittel wie Benzophenone-1, Benzophenone-3, Benzyl Salicylate, Etocrylene, Drometrizole, Butyl Methoxydibenzoylmethane und härtende Zusätze wie Formaldehyd und Hydrolysate aus Chitin und/oder Keratin. Auch antimykotische Zusätze sind möglich.

Die pflegenden Zusätze können in der erfindungsgemäßen Zusammensetzung beispielsweise in einer Menge von ≥ 0 und ≤ 5 Gew.-%, bezogen auf das Gesamtgewicht der Zusammensetzung, und vorzugsweise von ≥ 0,1 und ≤ 3 Gew.-% enthalten sein.

Bevorzugt enthält die erfindungsgemäße Nagellackzusammensetzung weiterhin Weichmacher und/oder effektgebende Bestandteile.

In einer bevorzugten Ausführungsform der Erfindung enthält die erfindungsgemäße Nagellackzusammensetzung eine wässrige Dispersion eines kationisch hydrophilierten Polyurethanharnstoffs, ≤ 10 Gew.-% an organischen Lösemitteln, bezogen auf die gesamte Nagellackzusammensetzung, effektgebende Bestandteile, Weichmacher und gegebenenfalls weitere Filmbildner und/oder in Nagellacken übliche Additive.

In einer weiterhin bevorzugten Ausführungsform der Erfindung enthält die Nagellackzusammensetzung
A) ≥ 0 und ≤ 10 Gew.-% an organischen Lösemitteln,
B) ≥ 0,1 und ≤ 60 Gew.-% des erfindungsgemäß verwendeten Polyurethanharnstoffs,
C) ≥ 10 und ≤ 95 Gew.-% an Wasser,
D) ≥ 0 und ≤ 40 Gew.-% an von B) verschiedenen Filmbildnern,
E) ≥ 0 und ≤ 20 Gew.-% an Weichmachern,
F) ≥ 0 und ≤ 30 Gew.-% an effektgebenden Bestandteilen,
G) ≥ 0 und ≤ 60 Gew.-% weitere in Nagellacken übliche Additive,
jeweils bezogen auf die Gesamtmasse der Nagellackzusammensetzung und wobei sich die Komponenten A) bis G) zu 100 Gew.-% ergänzen und wobei der Polyurethanharnstoff in einem Teil oder der ganzen Menge an Wasser dispergiert ist.

Besonders bevorzugt besteht die Nagellackzusammensetzung aus den Komponenten A) bis G).

Unter den weiteren in Nagellacken üblichen Additiven werden dabei insbesondere Koaleszenzmittel, Verdicker, Füllstoffe und/oder Nagelpflegende Zusätze verstanden.

In einer besonders bevorzugten Ausführungsform der Erfindung enthält die Nagellackzusammensetzung
A) ≥ 0 und ≤ 5 Gew.-% an organischen Lösemitteln,
B) ≥ 0,5 und ≤ 50 Gew.-% des erfindungsgemäß verwendeten Polyurethanharnstoffs,
C) ≥ 10 und ≤ 95 Gew.-% an Wasser,
D) ≥ 0 und ≤ 40 Gew.-% an von B) verschiedenen Filmbildnern,
E) ≥ 0,1 und ≤ 10 Gew.-% an Weichmachern,
F) ≥ 0,1 und ≤ 30 Gew.-% an effektgebenden Bestandteilen,
G) ≥ 0 und ≤ 60 Gew.-% weitere in Nagellacken übliche Additive, jeweils bezogen auf die Gesamtmasse der Nagellackzusammensetzung und wobei sich die Komponenten A) bis G) zu 100 Gew.-% ergänzen und wobei der Polyurethanharnstoff in einem Teil oder der ganzen Menge an Wasser dispergiert ist.

Insbesondere bevorzugt besteht die Nagellackzusammensetzung aus den Komponenten A) bis G).

Bei den erfindungsgemäßen Nagellackzusammensetzungen kann es sich auch um durch UV-Strahlung aushärtbare Systeme handeln. Bevorzugt handelt es sich jedoch um nicht UV-härtbare Systeme.

Die erfindungsgemäße Nagellackzusammensetzung ist bevorzugt abgefüllt in Gefäßen mit einem Füllvolumen von ≤ 50 ml, bevorzugt ≤ 20 ml.

Ein weiterer Gegenstand der Erfindung ist ein Verfahren zur Herstellung einer wässrigen Nagellackzusammensetzung, dadurch gekennzeichnet, dass eine wässrige Dispersion eines kationisch hydrophilierten Polyurethanharnstoffs und ≤ 10 Gew.-% an organischen Lösemitteln, bezogen auf die gesamte Nagellackzusammensetzung, eingesetzt werden. Die eingesetzten Rohstoffe werden dabei bevorzugt bei Raumtemperatur vermischt. Das Vermischen der Komponenten erfolgt dabei bevorzugt mit Hilfe einer Dissolverscheibe über einen Zeitraum von 5 bis 60 min.

Im Anschluss an das Vermischen werden die Komponenten bevorzugt mit einer Perlmühle über einen Zeitraum von 10 bis 120 min vermahlen.

Gegenstand der Erfindung ist auch ein Verfahren zur kosmetischen Beschichtung von Nägeln, dadurch gekennzeichnet, dass eine erfindungsgemäße wässrige Nagellackzusammensetzung auf Nägel aufgetragen wird.

Ebenfalls sind die durch das erfindungsgemäße Verfahren erhältlichen Beschichtungen bzw. Filme Gegenstand der Erfindung.

Unter Nägeln sind im Sinne dieser Erfindung Finger- und/oder Fußnägel, sowie künstliche Nägel zum Anbringen an Fingern oder Füßen zu verstehen.

Vorteilhaft verbleiben bei dem erfindungsgemäßen Verfahren die erfindungsgemäßen Nagellackzusammensetzungen zumindest teilweise auf den Nägeln.

Die Nagellackzusammensetzung wird mit einem Hilfsmittel auf die Finger- und/oder Fußnägel aufgetragen. Bei dem Hilfsmittel handelt es sich insbesondere um einen feinen Pinsel. Nach dem Auftragen bildet sich auf dem Nagel bevorzugt ein Film aus. Dieser Film härtet dann vorzugsweise aus. Bevorzugt härtet der Film dabei bei 23 °C innerhalb von ≤ 20 min, aus. Die Trocknungszeit bei Raumtemperatur wurde nach Auftrag der Zusammensetzung auf eine Glasplatte (120 µm nass) mittels eines Trocknungszeitbestimmungsgerätes "drying time recorder" der Fa. BYK Gardner GmbH, Deutschland gemäß ASTM D5895 bestimmt. Die Trocknungszeit "to the touch" wurde ermittelt.

Dabei kann die Aushärtung des Films ohne Hilfsmittel, aber auch unter zu Hilfenahme beispielsweise einer UV-Lampe erfolgen.

Die aus der erfindungsgemäßen Nagellackzusammensetzung erhältliche Beschichtung weist bevorzugt eine Pendelhärte von ≥ 40 König/s und ≤ 80 König/s, bevorzugt ≥ 50 König/s und ≤ 70 König/s auf. Die Pendelhärte wird dabei wie im Methodenteil der Beispiele angegeben bestimmt.

Des Weiteren weist die aus der erfindungsgemäßen Nagellackformulierung erhältliche Beschichtung bevorzugt einen Glanz ≥ 70 und ≤ 99, besonders bevorzugt von ≥ 80 und ≤ 98 auf. Die Bestimmung des Glanzes erfolgt dabei gemäß DIN 67530 mittels eines Glanzmesserätes "micro-haze plus" der Fa. BYK Gardner GmbH, Deutschland, nach Aufzug (200 µm nass) und Trocknung bei 23°C der jeweiligen Zusammensetzung nach 24 Stunden auf eine schwarz/weiß Leneta Karte. Der Glanz wird bei einem Winkel von 60° gemessen.

Weiterhin ist die aus der erfindungsgemäßen Nagellackformulierung erhältliche Beschichtung bevorzugt beständig gegen Wasser, das heißt insbesondere ist der erhaltene, ausgehärtete Beschichtungsfilm nach 4h in Wasser bei 40 °C noch vollständig und glatt und haftet noch auf dem Substrat auf das er aufgetragen wurde, besonders bevorzugt ist der Film dann noch vollständig, klar und haftet noch auf dem Substrat auf das er aufgetragen wurde. Die Wasserbeständigkeit wird dabei wie im Methodenteil der Beispiele angegeben bestimmt.

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.

Die Bestimmung der Festkörpergehalte (nicht-flüchtiger Anteil) erfolgte nach DIN-EN ISO 3251.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Das zahlenmittlere Molekulargewicht Mₙ wurde bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Die Glasübergangstemperatur T_{g} wurde mittels dynamischer Differenzkalorimetrie (DSC) in Anlehnung an die DIN EN 61006, Verfahren A, bestimmt, wobei ein DSC Gerät (Kalorimeter Pyris Diamond DSC von Perkin-Elmer) verwendet wird, das zur Bestimmung von T_{g} mit Indium und Blei kalibriert ist. Es werden 10 mg der zu untersuchenden Substanz in einen verschließbaren Aluminium-Tiegel eingewogen und dieser verschlossen. Es werden drei unmittelbar aufeinander folgende Durchläufe einer Aufheizungen von -100°C bis +150°C, Heizrate 20 K/min, mit anschließender Abkühlung Kühlrate 320 K/min vorgenommen und die dritte Aufheizkurve zur Bestimmung der Werte verwendet. Als Tg wird die Temperatur bei der halben Höhe einer Glasübergangsstufe bestimmt.

Die Bestimmung der Pendelhärten erfolgte an einem Pendelhärtemessgerät "König-Pendulum-Hardness-Tester" der Fa. BYK Gardner GmbH, Deutschland nach DIN EN ISO 1522, nach Aufzug (240 µm nass) und Trocknung der jeweiligen Zusammensetzung während der Nacht (16 Stunden) bei 23°C und 50% relativer Luftfeuchte auf eine Glasplatte. Es wurde jeweils der Mittelwert aus 3 Messungen gebildet.

Zur Messung der Wasserbeständigkeit wurden Glasplatten nach Auftrag der Zusammensetzung (240 µm) und 24 Stunden Trocknung bei 23°C und 50% relativer Luftfeuchte in ein Wasserbad bei 40°C über einen Zeitraum von 4 Stunden eingetaucht. Die Wasserbeständigkeit wurde qualitativ bestimmt.

### Verwendete Substanzen und Abkürzungen:

| | |
|---|---|
| Polyesterpolyol 1: | Polyesterpolyol aus Phthalsäureanhydrid und Ethylenglykol, Mₙ = 2000 g/mol, Tg = 21,5 °C, amorphe Struktur |
| Polyesterpolyol 2: | Polyesterpolyol aus Adipinsäure und Diethylenglykol, Mₙ = 2700 g/mol, Tg = -52,5°C, amorphe Struktur |

Die Polyesterpolyole und IPDI wurden hergestellt von der Bayer MaterialScience AG, Leverkusen, DE. Weitere Chemikalien von Sigma-Aldrich Chemie GmbH, Taufkirchen, DE. Die Rohstoffe wurden, soweit nicht anders erwähnt, ohne weitere Reinigung oder Vorbehandlung eingesetzt.

### Beispiel 1: Herstellung der Polyurethanharnstoffdispersion:

180,4 g einer Mischung aus Polyesterpolyol 1 und Polyesterpolyol 2 (Komponente b1) und b2) im Gewichtsverhältnis b1)/b2) = 1/2) wurden auf 100°C aufgeheizt und 1 h im Vakuum entwässert. Anschließend wurde abgekühlt und 51,2 g N-Methyldiethanolamin und 13,5 g 1,4-Butandiol zusammen mit 279,2 g Aceton zugegeben. Die Temperatur wurde auf 50 °C eingestellt und 212,3 g IPDI zugegeben und solange bei 50°C gerührt bis der theoretische NCO-Wert leicht unterschritten war. Das fertige Prepolymer wurde mit 41,2 g IPDA, welches gelöst in 150 g Aceton vorlag, bei 40°C umgesetzt. Die Nachrührzeit betrug 15 min. Danach wurde durch Zugabe von 232 g Wasser und 25,8 g 10%iger Essigsäure neutralisiert. Die Rührzeit betrug 30 min bei 50 °C. Anschließend wurde in 668,1 g Wasser bei 25 °C innerhalb von 15 min dispergiert. Es folgte die Entfernung des Lösemittels durch Destillation im Vakuum bei 40 °C und es wurde eine lagerstabile Dispersion erhalten.

| | |
|---|---|
| Feststoffgehalt: | 35% |
| Viskosität: | 89 mPas |

### Beispiele 2 bis 4: Anwendungsversuche

**Tabelle 1:**

| | Gew.% (bezogen auf die gesamte Zusammensetzung) | | |
|---|---|---|---|
| Beispiel | 2 | 3 | 4 |
| Styrene/Acrylate/ Ammonium Methacrylate Copolymer (Syn-tran^{®} PC 5620, Interpolymer, Feststoff: 42% in Wasser) | 77,9 | - | |
| Erfindungsgemäße Polyurethandispersion nach Beispiel 1 (Feststoff: 35 %) | - | 92,0 | |
| Anionisch hydrophilierte Polyurethanharn-stoffdispersion (Baycusan^{®} C 1004, Bayer MaterialScience AG) | | | 92,0 |
| Ammonium Hydroxide | 0,4 | - | |
| Red 7 Lake (25% dispersion in Syntran^{®} PC 5620, Interpolymer) | 9,9 | | |
| Red 7 Lake | - | 1,9 | 1,9 |
| Water (and) Simethicone, Sorbitan Stearate (and) Glyceryl Stearate(and) Polyglyceryl -10 Oleate (and) Cellulose Gum (50% in Wasser) | | 0,1 | 0,1 |
| Dipropylene Glycol Dibenzoate | 2,8 | 6,0 | 6,0 |
| Propylene Glycol n-Butyl Ether | 5,2 | - | |
| Ammonium Acrylates Copolymer (Syntran^{®} KL219-CG, Interpolymer) | 1,4 | - | |
| Sodium Magnesium Silicate and Tetrasodium Pyrophosphate (12,5% in Wasser) | 2,4 | - | |
| Pendelhärte [s] | 62±3s | 70±2s | 20 |
| Wasserbeständigkeit | Mäßig (körni-ger Film) | Sehr gute (ausgezeichnete Filmqualität) | Schlecht (Film ist abgelöst vom Substrat und nicht mehr glatt, sondern faltig) |

### Herstellung der Nagellackzusammensetzung:

Die in der Tabelle 1 aufgeführten Rohstoffe wurden mit Hilfe einer Dissolverscheibe 30 min lang bei 23 °C und 2200 U/min vermischt. Anschließend wurde der Rohstoffmix 45 min lang bei 23 °C in einer Perlmühle der Firma Vollrath (Dissolverscheibe Ø 75 mm, Geschwindigkeit: 2865 U/min, Glasperlen Ø 2,85-3,45 mm, Masse Formulierung = Masse Glasperlen) vermahlen. Die so erhaltene Nagellackzusammensetzung wurde nach den im Methodenteil angegeben Verfahren hinsichtlich ihrer Härte und Wasserbeständigkeit untersucht.

Aus den Beispielen 2 bis 4 ist deutlich ersichtlich, dass die erfindungsgemäß verwendeten, wässrigen, kationisch hydrophilierten Polyurethanharnstoffdispersionen gegenüber den nach dem Stand der Technik bekannten Filmbildnern für wässrige Nagellackzusammensetzungen eine deutlich verbesserte Härte und auch Wasserbeständigkeit aufweisen.

## Patentansprüche

1. Wässrige Nagellackzusammensetzung, **dadurch gekennzeichnet, dass** diese eine wässrige Dispersion eines kationisch hydrophilierten Polyurethanharnstoffs und ≤ 10 Gew.-% an organischen Lösemitteln, bezogen auf die gesamte Nagellackzusammensetzung, enthält.

2. Nagellackzusammensetzung gemäß Anspruch 1, **dadurch gekennzeichnet, dass** der Po-lyurethanharnstoff einen Gehalt an kationischen und/oder potentiell kationischen Gruppen von ≥ 0,2 und ≤ 5 Milliequivalenten pro g Polymer aufweist.

3. Nagellackzusammensetzung gemäß Anspruch 1 oder 2, **dadurch gekennzeichnet, dass** der Polyurethanharnstoff aufgebaut ist aus
a) wenigstens einem aliphatischen, araliphatischen und/oder cycloaliphatischen Polyisocyanat,
b) wenigstens einem Polyol mit einem zahlenmittleren Molekulargewicht Mn ≥ 400 und ≤ 6000 g/mol und einer Hydroxylfunktionalität von ≥ 1,5 und ≤ 4,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist.

4. Nagellackzusammensetzung gemäß Anspruch 3, **dadurch gekennzeichnet, dass** der Po-lyurethanharnstoff erhältlich ist durch Umsetzung der Komponenten a), b), c) und gegebenenfalls e) zu einem Isocyanat-terminierten Präpolymer, anschließender Umsetzung des Präpolymers mit der Komponente d) und gegebenenfalls den Komponenten e) und f) und falls die Komponente d) eine potentiell kationische Gruppe umfasst, Neutralisation dieser Gruppe durch eine Säure vor, während oder nach der Umsetzung des Isocyanat-terminierten Präpolymers mit der Komponente d) und gegebenenfalls den Komponenten e) und f).

5. Nagellackzusammensetzung gemäß Anspruch 3 oder 4, **dadurch gekennzeichnet, dass** die Komponente b) ausgewählt ist aus Polyesterpolyolen.

6. Nagellackzusammensetzung gemäß einem der Ansprüche 3 bis 5, **dadurch gekennzeichnet, dass** die Komponente b) wenigstens ein Polyesterpolyol umfasst, das als Aufbaukomponente wenigstens eine aromatische Dicarbonsäure oder das entsprechende Carbonsäureanhydrid enthält.

7. Nagellackzusammensetzung gemäß einem der Ansprüche 3 bis 6, **dadurch gekennzeichnet, dass** die Komponente b) wenigstens ein Polyesterpolyol b1) umfasst, das als Aufbaukomponente wenigstens einen Anteil an aromatischen Dicarbonsäuren und/oder den entsprechenden Carbonsäureanhydriden von ≥ 60 Gew.-%, bezogen auf das Gesamtgewicht des Polyesterpolyols b1), enthält.

8. Nagellackzusammensetzung gemäß Anspruch 7, **dadurch gekennzeichnet, dass** die Komponente b) neben dem Polyesterpolyol b1) ein weiteres Polyesterpolyol b2) umfasst, das bevorzugt als Aufbaukomponente wenigstens eine aliphatische Dicarbonsäure und/oder das entsprechende Carbonsäureanhydrid enthält.

9. Nagellackzusammensetzung gemäß Anspruch 8, **dadurch gekennzeichnet, dass** die Komponente b) aus einem Polyesterpolyol b1), das als Säurekomponente ausschließlich aromatische Dicarbonsäuren und/oder die korrespondierenden Anhydride enthält und einem Polyesterpolyol b2), das als Säurekomponente aliphatische Dicarbonsäuren und/oder die korrespondierenden Anhydride enthält, besteht, wobei b1) eine amorphe Struktur aufweist.

10. Nagellackzusammensetzung gemäß einem der Ansprüche 3 bis 9, **dadurch gekennzeichnet, dass** die Komponente c) wenigstens eine tertiäre Aminogruppe und/oder eine Ammoniumgruppe aufweist.

11. Nagellackzusammensetzung gemäß einem der Ansprüche 1 bis 10, **dadurch gekennzeichnet, dass** diese ≤ 5 Gew.-% an organischen Lösemitteln, bezogen auf die Gesamtmasse der Nagellackzusammensetzung, enthält.

12. Nagellackzusammensetzung gemäß einem der Ansprüche 1 bis 11, **dadurch gekennzeichnet, dass** diese weiterhin Weichmacher und/oder effektgebende Bestandteile aufweist.

13. Verfahren zur Herstellung einer wässrigen Nagellackzusammensetzung, **dadurch gekennzeichnet, dass** eine wässrige Dispersion eines kationisch hydrophilierten Polyurethanharn-stoffs und ≤ 10 Gew.-% an organischen Lösemitteln, bezogen auf die gesamte Nagellackzusammensetzung, eingesetzt werden.

14. Verwendung von wässrigen Dispersionen von kationisch hydrophilierten Polyurethanharn-stoffen in wässrigen Nagellackzusammensetzungen, die ≤ 10 Gew.-% an organischen Lösemitteln, bezogen auf die gesamte Nagellackzusammensetzung, enthalten.

15. Verfahren zur kosmetischen Beschichtung von Nägeln, **dadurch gekennzeichnet, dass** eine wässrige Nagellackzusammensetzung gemäße einem der Ansprüche 1 bis 12 auf Nägel aufgetragen wird.
